(19) Europäisches Patentamt
European Patent Office
Office européen des brevets

(11) Publication number: 0 145 095 A2

# (12) EUROPEAN PATENT APPLICATION

(21) Application number: 84201800.4

(22) Date of filing: 05.12.84

(51) Int. Cl.[4]: C 07 D 285/08
C 07 D 271/06, C 07 D 417/12
C 07 D 413/12, A 01 N 43/82

(30) Priority: 06.12.83 JP 230384/83
31.05.84 JP 109643/84
31.05.84 JP 109644/84
31.05.84 JP 109645/84
31.05.84 JP 109646/84
08.08.84 JP 164856/84
08.08.84 JP 164857/84

(43) Date of publication of application:
19.06.85 Bulletin 85/25

(84) Designated Contracting States:
AT BE CH DE FR GB IT LI NL

(71) Applicant: NIPPON SODA CO., LTD.
2-1, Ohte-machi 2-chome
Chiyoda-ku, Tokyo, 100(JP)

(72) Inventor: Hagiwara, Kenji
16-5, Nakasato
Odawara-shi Kanagawa-ken(JP)

(72) Inventor: Hara, Tamio
2-6-23, Koma
Ohiso-machi Naka-gun Kanagawa-ken(JP)

(72) Inventor: Yamada, Tomio
28-3, Kurobeoka
Hiratsuka-shi Kanagawa-ken(JP)

(72) Inventor: Takahashi, Hidemitsu
420-2, Nagamochi
Hiratsuka-shi Kanagawa-ken(JP)

(72) Inventor: Hatano, Renpei
2-6-23, Koma, Ohiso-machi
Naka-gun Kanagawa-ken(JP)

(74) Representative: van der Beek, George Frans et al,
Nederlandsch Octrooibureau Johan de Wittlaan 15 P.O. Box 29720
NL-2502 LS 's-Gravenhage(NL)

## (54) 1,2,4-Oxa (Thia) diazolin-3-one derivatives.

(57) A compound having the formula:

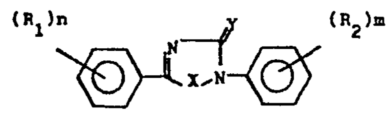

wherein each of X and Y is oxygen or sulfur;

$R_1$ is same or different substituent(s) selected from the group consisting of halogen, $C_{1-3}$ haloalkyl and $C_{1-3}$ alkoxy;

$R_2$ is same or different substituent selected from the group consisting of halogen, saturated or unsaturated $C_{1-6}$ hydrocarbon (which may be substituted by halogen), $C_{1-6}$ alkoxycarbonyl, $C_{1-6}$ alkyl substituted amino, phenylazo (which may be substituted by $C_{1-6}$ alkyl) and $-A-R$ wherein A is oxygen or sulfur, and

R is saturated or unsaturated $C_{1-6}$ hydrocarbon (which may be substituted by halogen, phenyl and/or phenoxy), or aryl containing not more than ten members and two or less thereof may be nitrogen (which may be substituted by halogen, cyano, $C_{1-6}$ haloalkyl and/or nitro);

n is zero or an integer of 1 to 3; and

m is an integer of 1 to 5.

and an insecticide and/or fungicide containing said compound.

EP 0 145 095 A2

SPECIFICATION

# 1,2,4-Oxa (Thia) diazolin-3-one Derivatives

The present invention relates to 1,2,4-oxa (thia) diazolin-3-one derivatives, insecticidal and/or agricultural fungicidal compositions in the form of mixture of such compound(s) with inert carrier(s), and a process for the preparation of such compounds.

According to a first aspect of the present invention, there is provided a compound having the formula:

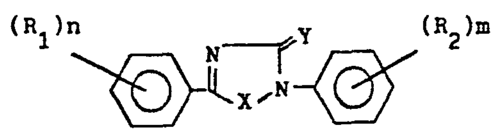

..... (I)

wherein each of X and Y is oxygen or sulfur;

$R_1$ is same or different substituent(s) selected from the group consisting of halogen, $C_{1-3}$ alkyl, $C_{1-3}$ haloalkyl and $C_{1-3}$ alkoxy;

$R_2$ is same or different substituent selected from the group consisting of halogen, saturated or unsaturated $C_{1-6}$ hydrocarbon (which may be substituted by halogen), $C_{1-6}$ alkoxycarbonyl, $C_{1-6}$ alkyl substituted amino, phenylazo (which may be substituted by $C_{1-6}$ alkyl) and -A-R wherein

A is oxygen or sulfur, and

R is saturated or unsaturated $C_{1-6}$ hydrocarbon (which may be substituted by halogen, phenyl and/or phenoxy), or aryl containing not more than ten members and two or less thereof may be nitrogen (which may be substituted by halogen, cyano, $C_{1-6}$ haloalkyl and/or nitro);

n is zero or an integer of 1 to 3; and

m is an integer of 1 to 5.

According to a second aspect of the present invention there is provided an insecticidal and/or agricultural fungicidal composition comprising an inert carrier and an effective amount of the compound having the formula (I).

A process for the preparation of 2-phenyl-5-phenyl-1,2,4-oxa (thia)-diazolin-3-one derivatives which are similar to the compounds of the present invention, is disclosed in Chemisch Berichte 1966 99 (3) P 782-792, ibid 1973 106 (5) P 1496-1500, and Heterocycles 1976 5 (1) P 189-194.

However, in those literatures, any compound having substituent on phenyl radical at the 2-position is not described and further any research on biological activity is not shown even with said non substituted phenyl compound.

A large number of insecticidal chemicals have been used and organophosphrous compounds or carbamates are the most typical chemicals.

Insects resistant against those chemicals, however, have become to appear and increase. Therefore, the control of such resistant insects has become difficult.

Resistant insects against synthesized pyrethroides, for which we have very big concern, have been reported recently.

Accordingly, new insecticides having a structure basically different from conventional insecticides are highly expected to appear in the market.

Also, appearance of agricultural insecticides with highly fungicidal effect has been awaited.

The compound having the formula (I) has a superior insecticidal activity and further, has a broader insecticidal spectrum and indicates a superior insecticidal activity on various kinds of injurious insects in Lepidoptera species, Coleoptera species, Diptera species or the like.

Furthermore, the compound has not only a larvicidal activity and an ovicidal activity but also an ovicidal effect on ovum under oviposition in case of treating the adult insect with the compound.

The compound having the formula (I) further has a fungicidal activity.

The compound has a low toxicity to haematothermal animals and can be used in safety.

According to a third aspect of the invention, there is provided a process for the preparation of the compound of formula (I), comprising the step of reaction as illustrated by the following equations:

(1) In case that X is sulfur and Y is oxygen:

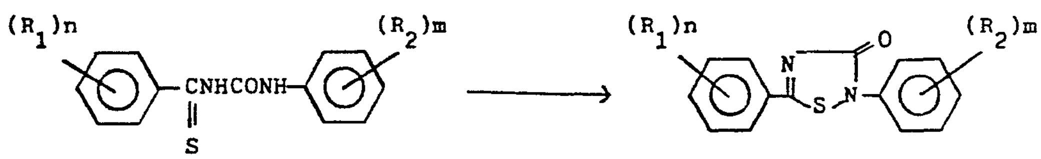

(II) (I)'

To produce the compound having formula (I)', thiobenzoylurea having the formula (II) is reacted with an oxidizing agent such as bromine, chlorine, sodium hypochlorite and sodium hypobromite etc. in an inert solvent, for example, dichloromethane, chloroform, N,N-dimethylformamide, dialkylketones, such as methyl isobutyl ketone, methyl ethyl ketone and acetone, dialkyl ethers such as diethylether and dimethylether, cyclic ethers such as tetrahydrofuran and dioxane and ethylacetates such as ethylacetate and methylacetate in the presence of a base, for example, amines, desirably, tertiary amines, such as trimethyl amine, triethyl amine, dimethylaniline, diethylaniline, pyridines etc., and castic alkali such as example, caustic soda and caustic potasium for a time less than 2 hours, desirably for 30 to 60 minutes, at a temperature lower than 60°C, desirably from 0°C to room temperature.

The compound having the formula (I)' thus produced may be obtained by a usual procedure of separation and then purified by a conventional purifying

procedure such as recrystallization, column chromatography etc.

The starting material having the formula (II) can be prepared by the following equation:

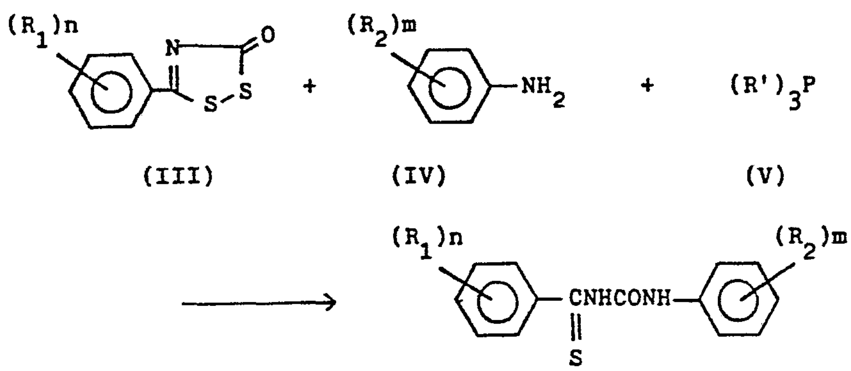

(III) (IV) (V)

(II)

(In the formula (V), R' is alkyl or aryl with or without substituent(s))

Depending on the type of the substituents of $(R_1)n$ and $(R_2)m$, the compounds (I)' may also be prepared by the following equation:

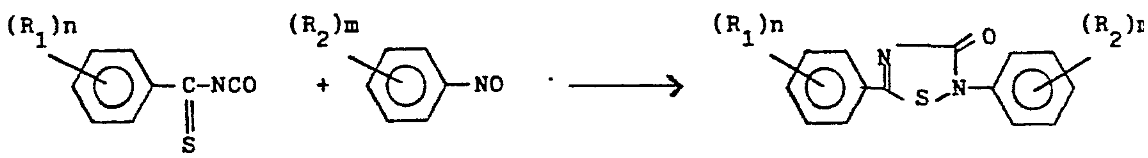

(VI) (VII) (I)'

(2) In case that X and Y are oxygen:

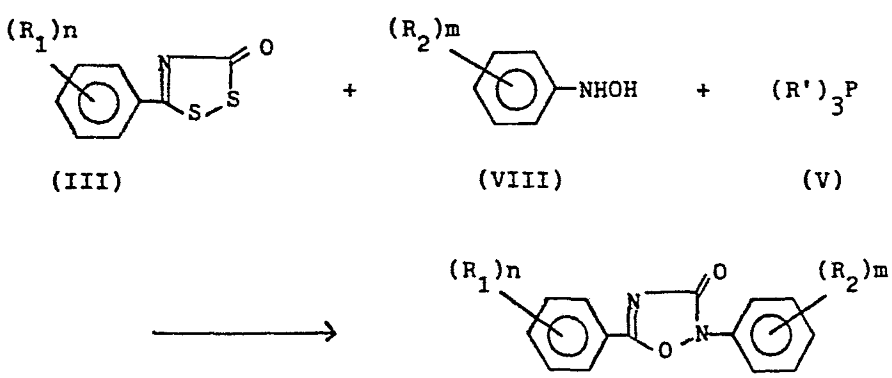

(III) (VIII) (V)

(I)"

The reaction is carried out in an inert solvent for example dichloromethane, chloroform, tetrahydrofuran, benzene, toluene, N,N-dimethylformamide, dialkylketones such as acetone, methylethylketone and methyisobutylketone, alkylacetate such as ethylacetate and methylacetate, and cyclic ethers such as tetrahydrofuran and dioxane in the presence of equivalent amount of trivalent phosphorus compound (V) such as trialkyl phosphine, triphenyl phosphine etc. at a temperature from -20°C to 50°C.

This process gives better yields, of $\Delta^4$-1,2,4-oxadiazolin-3-ones compared with the process reported by J. Goerdeler and R. Schimpf Chem. Ber 106 1496 (1973), when $(R_1)n$ represents the substituents at 2-position or 2,6-position on the benzene ring.

The compound having the formula (I)" thus produced may be obtained by the usual procedure of separation and then purified by a conventional procedure such as recrystallization, column chromatography etc.

The starting N-arylhydroxylamines (VIII) can be produced by reduction of corresponding nitro compounds with zinc.

When $(R_2)m$ represents the substituents at 2-position on the benzene ring, the crude extracts containing N-arylhydroxylamines are employed as starting materials without further purification because such N-arylhydroxylamines are too unstable to isolate.

(3) In case that X is oxygen or sulfur and Y is sulfur:

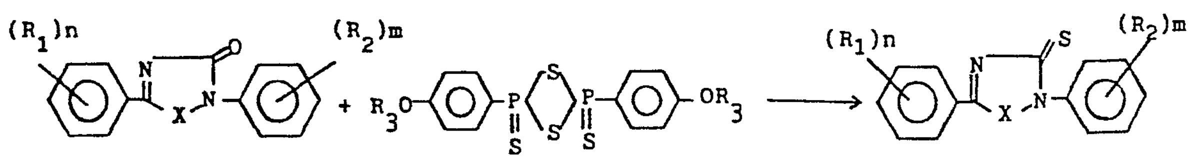

(I)'or (I)" (IX) (I)"'

(In the formula (IX), $R_3$ is $C_{1-6}$ alkyl)

The compound having the formula (I)' or (I)" is reacted with Lawesson's Reagent having the formula (IX) in an inert solvent, for example, dimethoxy ethane, toluene etc. The reaction temperature is normally from 50 to 100°C.

The molar ratio of the compound having the formula (I)' or (I)" to Lawesson's Reagent is preferred to be from 2:1 to 1:5.

The compound having the formula (I)"' thus produced may be obtained by usual procedure of separation and then purified by a conventional purifying procedure such as recrystallization, column chromatography etc.

A chemical structure of the obtained compound was determined by means of NMR spectrum, Mass spectrum and IR spectrum.

The following Examples illustrate the invention.

Example 1 : 2-(4-chlorophenyl)-5-(2,6-difluorophenyl)-$\Delta^4$-1,2,4-thiadiazolin-3-one (Compound No. 26)

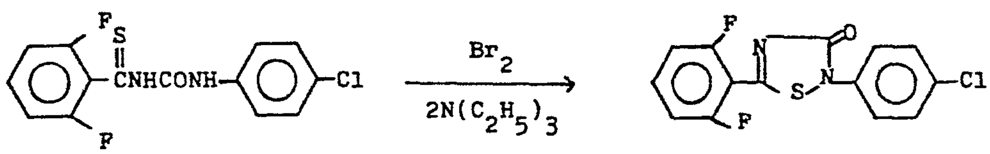


A sulution of 0.6 g of bromine in 5 ml of dichloromethane was added dropwise to a solution of 1.3 g of 1-(4-chlorophenyl)-3-(2,6-difluorothiobenzoyl) urea and 0.8 g of triethylamine in 30 ml of dichloromethane keeping at 0 to 5°C under stirring. The mixture was stirred at room temperature for 30 minutes and then the reaction mixture was washed with 20 ml of water. The washed solution was dried over anhydrous magnesium sulfate and concentrated under reduced pressure. The residue was washed with ethanol to obtain 0.9 g of desired product. m.p. 170 - 172°C.

Example 2 : 2-(3,5-dichloro-4-(3-chloro-5-trifluoromethyl-2-pyridyloxy)phenyl)-5-(2,6-difluorophenyl)-$\Delta^4$-1,2,4-thiadiazolin-3-one (Compound No. 107)

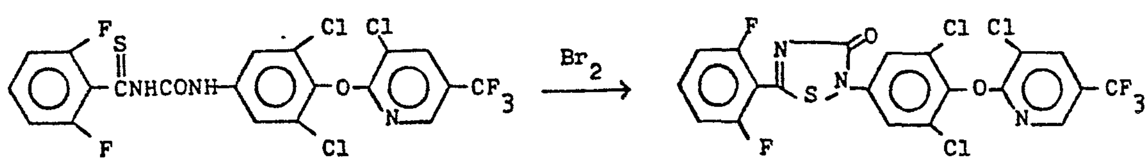

A solution 0.5 g of bromine in 5 ml of dichloromethane was added dropwise to a solution of 1.7 g of 1-(3,5-dichloro-4-(3-chloro-5-trifluoromethyl-2-pyridyloxy)phenyl)-3-(2,6-difluorothiobenzoyl) urea and 0.7 g of triethylamine in 30 ml of dichloromethane under ice cooling and stirring. The mixture was stirred for 30 minutes and the reaction mixture was washed with 50 ml of water. The washed solution was dried over anhydrous magnesium sulfate and then concentrated under reduced pressure. The residue was washed with a mixed solvent (dichloromethane: n-hexane=1:1 v/v) to obtain 0.85 g of desired product. m.p. 204 - 210°C.

Example 3: 5-(2-chlorophenyl)-2-(4-trifluoromethoxyphenyl)-$\Delta^4$-1,2,4-thiadiazoline-3-thione (Compound No. 214)

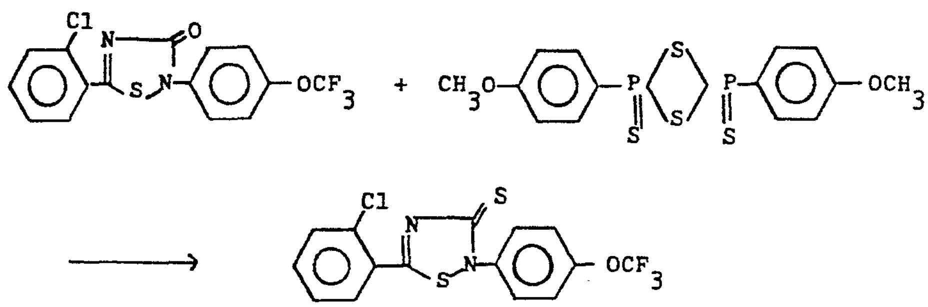

To a suspension of 5-(2-chlorophenyl)-2-(4-trifluoromethoxyphenyl)-$\Delta^4$-1,2,4-thiadiazolin-3-one (3.7 g) in dry dimethoxyethane (40 ml) was added Lawesson's Reagent (2.1 g) and the resultant mixture was heated at 80°C for 10 minutes. Upon the time all the crystals have dissolved, dimethoxyethane was removed in vacuo and the residue was dissolved in chloroform (40 ml). The chloroform solution was washed with a 5% aqueous sodium hydroxide solution and water in order and then dried over anhydrous magnesium sulfate and filtrated. The filtrate was concentrated under reduced pressure, and the residue was purified by silica gel column chromatography to give 1.9 g of 5-(2-chlorophenyl)-2-(4-trifluoromethoxyphenyl)-$\Delta^4$-1,2,4-thiadiazoline-3-thione. m.p. 75 - 77°C.

Example 4: 2-(4-chlorophenyl)-5-(2,6-difluorophenyl)-$\Delta^4$-1,2,4-oxadiazolin-3-one (Compound No. 256)

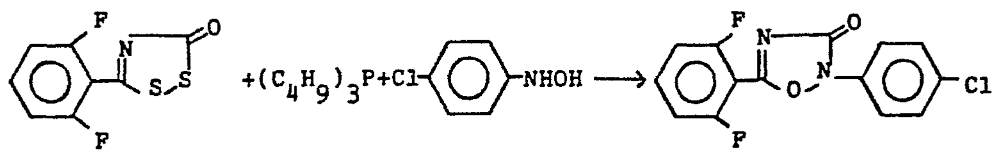


A solution of N-(4-chlorophenyl) hydroxylamine (0.75 g) and tributyl phosphine (1.05 g) in dichloromethane (60 ml) was added dropwise to a solution of 5-(2,6-difluorophenyl)-1,2,4-dithiazol-3-one (1.2 g) in dichloromethane (60 ml) keeping at 0 to 10°C. The reaction mixture was stirred at room temperature for an hour and concentrated under reduced pressure. The residue was recrystallized from dioxane to obtain 0.8 g of 2-(4-chlorophenyl)-5-(2,6-difluorophenyl)-$\Delta^4$-1,2,4-oxadiazolin-3-one. m.p. 178 - 181°C.

Example 5 : 2-(2,5-dichloro-3-trifluoromethylphenyl)-5-(2,6-difluorophenyl)-$\Delta^4$-1,2,4-oxadiazolin-3-one (Compound No. 244)

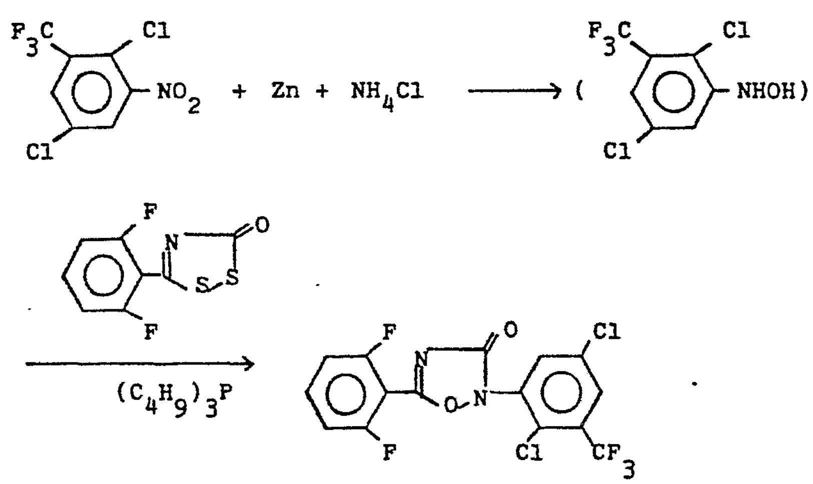


A solution of ammonium chloride (0.3 g) in water (2 ml) was added dropwise to a suspension of 2,5-dichloro-3-trifluoromethylnitrobenzene (1.45 g) and zinc dust (0.75 g) in hot ethanol (5 ml) at such a rate to maintain gentle reflux. When the exothermic reaction had ceased, the reaction mixture was filtered and the filtrate was extracted with 50 ml of chloroform. The chloroform layer was washed twice with 50 ml of water, dried over anhydrous magnesium sulfate and filtered to obtain a crude solution of N-(2,6-dichloro-3-trifluoromethylphenyl) hydroxylamine in chloroform. To this solution was added 5-(2,6-difluorophenyl)-1,2,4-dithiazol-3-one (1.1 g) and the resultant mixture was cooled to 0 - 10°C, then tributyl phosphine (1 g) was added thereto. The reaction mixture was stirred for an hour and then concentrated under reduced pressure. The residue was purified by silica-gel column chromatography to obtain 0.25 g of 2-(2,5-dichloro-3-trifluoromethylphenyl)-5-(2,6-difluorophenyl)-$\Delta^4$-1,2,4-oxadiazolin-3-one. m.p. 143 - 145°C.

Inclusive of the above, each compound within the scope of this invention which can be prepared in analogeous method(s) is tabulated in Table 1.

Table 1

| Compound No. | Structural Formula | | | | Physical Properties ( ) m.p. °C |
|---|---|---|---|---|---|
| | 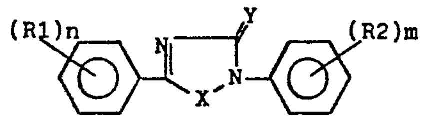 | | | | |
| | X | Y | (R1)n | (R2)m | |
| 1 | S | O | 2-Cl | 4-SCF3 | 165-167 |
| 2 | " | " | 2,6-F2 | " | 178-180 |
| 3 | " | " | " | 4-O-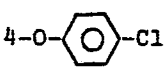-Cl | 178-180 |
| 4 | " | " | 2-Cl | 4-O-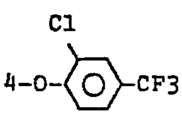-CF3 | 175-177 |
| 5 | " | " | 2,6-F2 | " | 156-158 |
| 6 | " | " | 2-F | 4-OCF3 | 134-135 |
| 7 | " | " | 2-Cl 6-F | " | 60-64 |
| 8 | " | " | 2-Cl | " | 165-167 |
| 9 | " | " | 2-CH3 | " | 119-121 |
| 10 | " | " | 2-CF3 | " | 100-102 |
| 11 | " | " | 2-Br | " | 154-155 |
| 12 | " | " | 2,6-F2 | " | 138-142 |
| 13 | " | " | 2,6-Cl2 | " | 139-141 |
| 14 | " | " | 2-Cl | 4-OCF2CFHCF3 | 181-182 |
| 15 | " | " | " | 4-OCF2CFClH | 193-195 |
| 16 | " | " | 2,6-F2 | " | 168-169 |
| 17 | " | " | 2-Cl | 4-OCF2CF2H | 176-180 |
| 18 | " | " | 2,6-F2 | " | 153-156 |

| | | | | | |
|---|---|---|---|---|---|
| 19 | S | O | 2,6-F2 | 4-O- 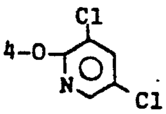 | 190-192 |
| 20 | " | " | 2-Cl | 4-N=N- 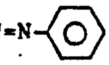 | 229-230 |
| 21 | " | " | 2,6-F2 | " | 203-204 |
| 22 | " | " | H | 4-F | 179-182 |
| 23 | " | " | 4-Cl | 4-Cl | 234-236 |
| 24 | " | " | 4-CH3 | " | 210-213 |
| 25 | " | " | 2-Cl | " | 203-205 |
| 26 | " | " | 2,6-F2 | " | 170-172 |
| 27 | " | " | 2,6-Cl2 | " | 150-154 |
| 28 | " | " | 2-Cl | 4-COOC4H9t | 113-114 |
| 29 | " | " | 2-CH3 | " | 181-183 |
| 30 | " | " | 2,6-F2 | " | 195-196 |
| 31 | " | " | H | 4-CH3 | 144-146 |
| 32 | " | " | " | 4-CF3 | 212-214 |
| 33 | " | " | 2,6-F2 | " | 184-187 |
| 34 | " | " | 2,6-Cl2 | " | 158-161 |
| 35 | " | " | 2-Cl | 4-C≡CH | 172-173 |
| 36 | " | " | 2,6-F2 | " | 200-202 |
| 37 | " | " | 2-Cl | 4-C3H7n | 143-145 |
| 38 | " | " | 2,6-F2 | " | 142-145 |
| 39 | " | " | H | 4-Br | 187-189 |
| 40 | " | " | 2-Cl | 3-OCF3 4-Cl | 165-167 |
| 41 | " | " | 2-CH3 | " | 98-99 |
| 42 | " | " | 2,6-F2 | " | 123-124 |
| 43 | " | " | 2-Cl | 3-OCF3 | 133-134 |
| 44 | " | " | 2,6-F2 | 3-OCF2CF2H 4-COOC2H5 | 116-118 |
| 45 | " | " | " | 3-OC2H5 4-CF3 | 147-148 |

| | | | | | |
|---|---|---|---|---|---|
| 46 | S | O | 2-Cl | 3-O- 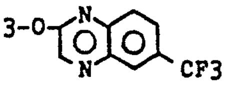 | 175-176 |
| 47 | " | " | 2,6-F2 | " | 193-195 |
| 48 | " | " | 2-Cl | 3-O- 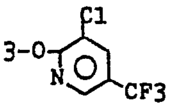 | 173-174 |
| 49 | " | " | 2,6-F2 | " | 159-161 |
| 50 | " | " | " | 3-F 4-OCF3 | 125-126 |
| 51 | " | " | " | 3-Cl 5-CF3 | 155-157 |
| 52 | " | " | 2-Cl | 3-Cl 4-O- 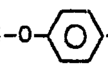 -CN | 250 up |
| 53 | " | " | 2,6-F2 | " | 202-206 |
| 54 | " | " | 2-F | 3-Cl 4-OCF3 | 162-163 |
| 55 | " | " | 2-Cl | " | 178-183 |
| 56 | " | " | 2-CH3 | " | 139-140 |
| 57 | " | " | 2,6-F2 | " | 154-157 |
| 58 | " | " | 2-Cl | 3-Cl 4-OCF2CF2H 5-Br | 153-155 |
| 59 | " | " | 2,6-F2 | " | 151-152 |
| 60 | " | " | 2-Cl | 3-Cl 4-OCF2CF2H | 169-174 |
| 61 | " | " | 2-CH3 | " | 124-127 |
| 62 | " | " | 2,6-F2 | " | 183-187 dec. |
| 63 | " | " | 2-F | 3-Cl 4-CF3 | 174-175 |
| 64 | " | " | 2-Cl 6-F | " | 134-136 |
| 65 | " | " | 2-Cl | " | 183-186 |
| 66 | " | " | 2-CH3 | " | 147-148 |
| 67 | " | " | 2-Br | " | 199-200 |
| 68 | " | " | 2,6-F2 | " | 162-164 |
| 69 | " | " | 2-Cl | 3-CH3 4-OCF2CF2H | 143-147 |
| 70 | " | " | 2,6-F2 | " | 151-156 |

0145095

| | | | | | |
|---|---|---|---|---|---|
| 95 | S | O | 2,6-F2 | 3,5-Cl2 4-OCH2C≡CH | 214-216 |
| 96 | " | " | 2-Cl 6-F | 3,5-Cl2 4-OCF2CFClH | 136-137 |
| 97 | " | " | 2-Cl | " | 145-148 |
| 98 | " | " | 2,6-F2 | " | 152-155 |
| 99 | " | " | 2-Cl | 3,5-Cl2 4-OCF2CF2H | 139-140 |
| 100 | " | " | 2-CH3 | " | 120-122 |
| 101 | " | " | 2-Br | " | 170-171 |
| 102 | " | " | 2,6-F2 | " | 158-160 |
| 103 | " | " | 2-CH3 | 3,5-Cl2 4-OCF2CClFH | 143-145 |
| 104 | " | " | 2,6-F2 | 3,5-Cl2 4-OCF2CCl2H | 182-186 |
| 105 | " | " | " | 3,5-Cl2 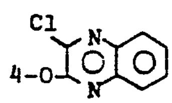 | 237-238 |
| 106 | " | " | 2-Cl | 3,5-Cl2 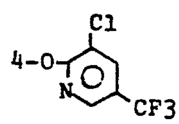 | 203-208 |
| 107 | " | " | 2,6-F2 | " | 204-210 |
| 108 | " | " | 2-Cl | 3,5-Cl2 4-F | 263-264 |
| 109 | " | " | 2,6-F2 | " | 237-240 |
| 110 | " | " | H | 3,5-Cl2 | 186-187 |
| 111 | " | " | 2-Cl | 3,5-(CH3)2 4-OCF3 | 154-158 |
| 112 | " | " | 2-CH3 | " | 110-118 |
| 113 | " | " | 2,6-F2 | " | 168-163 |
| 114 | " | " | 2-Cl | 3,5-(CH3)2 4-OCF2CF2H | 158-160 |
| 115 | " | " | 2,6-F2 | " | 143-145 |
| 116 | " | " | 2,6-Cl2 | 3,4-Cl2 | 172-177 dec. |
| 117 | " | " | 2,6-F2 | 3,4,5-Cl3 | 236 dec |
| 118 | " | " | 2-Cl | 2-F 4-N(CH3)2 5-CF3 | 152-156 |
| 119 | " | " | 2,6-F2 | " | 155-157 |

| | | | | | |
|---|---|---|---|---|---|
| 71 | S | O | 2-Cl | 3-CF3 4-OCF2CFHCF3 | 144-146 |
| 72 | " | " | 2,6-F2 | " | 133-134 |
| 73 | " | " | 2-Cl | 3-CF3 4-F | 184-187 |
| 74 | " | " | 2,6-F2 | " | 203 |
| 75 | " | " | 2-Cl | 3-CF3 4-Cl | 206 dec. |
| 76 | " | " | 2-CH3 | " | 149-151 |
| 77 | " | " | 2,6-F2 | " | 175-177 |
| 78 | " | " | 2-Cl | 3-CF3 4-Br | 221-224 |
| 79 | " | " | 2-CH3 | " | 139-141 |
| 80 | " | " | 2,6-F2 | " | 184-188 |
| 81 | " | " | 2-Cl | 3-Br 4-Cl 5-CF3 | 201-204 |
| 82 | " | " | 2,6-F2 | " | 169-172 |
| 83 | " | " | 2-Cl | 3,5-Cl2 4-SCH2CH=CHCl | 148-150 |
| 84 | " | " | 2,6-F2 | " | 167-168 |
| 85 | " | " | 2-Cl | 3,5-Cl2 4-O-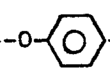-NO2 | 221-223 |
| 86 | " | " | 2,6-F2 | " | 222-225 |
| 87 | " | " | " | 3,5-Cl2 4-O-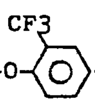-NO2 | 201-204 |
| 88 | " | " | 2-Cl | 3,5-Cl2 4-OCH2CH=CHCl | 179-181 |
| 89 | " | " | 2,6-F2 | " | 188-192 dec. |
| 90 | " | " | 2-Cl | 3,5-Cl2 4-OCH2CH2-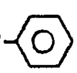 | 181-185 |
| 91 | " | " | 2,6-F2 | " | 182-184 |
| 92 | " | " | 2-Cl | 3,5-Cl2 4-OCH2CH2O-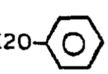 | 193-194 |
| 93 | " | " | 2,6-F2 | " | 177-180 |
| 94 | " | " | 2-Cl | 3,5-Cl2 4-OCH2C≡CH | 237-238 |

| | | | | | |
|---|---|---|---|---|---|
| 120 | S | O | 2-Cl | 2-F 4-Cl 5-O-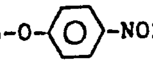-NO2 | 208-209 |
| 121 | " | " | 2,6-F2 | " | 187-190 |
| 122 | " | " | " | 2-F 4-Cl 5-OCH2C≡CH | 187-189 |
| 123 | " | " | " | 2-F 4-Cl | 203-204 |
| 124 | " | " | 2-Cl | 2-F 4-Br | 220-225 dec. |
| 125 | " | " | 2,6-F2 | " | 219-220 |
| 126 | " | " | 2-Cl | 2-F 4,5-Cl2 | 231-232 |
| 127 | " | " | 2,6-F2 | " | 167-168 |
| 128 | " | " | 2-F | 2-F 3-CF3 5-Cl | 141-143 |
| 129 | " | " | 2-Cl | " | 163-166 |
| 130 | " | " | 2-CH3 | " | 117-118 |
| 131 | " | " | 2,6-F2 | " | 173-175 |
| 132 | " | " | " | 2-Cl 5-OCF3 | 124-125 |
| 133 | " | " | 2-Cl | 2-Cl 4-OCF3 | 183-185 |
| 134 | " | " | 2-CH3 | " | 118-119 |
| 135 | " | " | 2,6-F2 | " | 137-138 |
| 136 | " | " | 2-Cl | 2-Cl 4-F 5-OCF3 | 220-222 |
| 137 | " | " | 2,6-F2 | " | 168-170 |
| 138 | " | " | 2-Cl | 2-Cl 4-F | 167-169 |
| 139 | " | " | 2,6-F2 | " | 172-173 |
| 140 | " | " | 2-Cl | 2-Cl 4-CF3 | 195-199 |
| 141 | " | " | 2-CH3 | " | 140-143 |
| 142 | " | " | 2,6-F2 | " | 171-172 |
| 143 | " | " | 2-Cl | 2-Cl 3-CF3 5-Br | 203-204 |
| 144 | " | " | 2,6-F2 | " | 187-188 |
| 145 | " | " | 2-Cl | 2-CH3 4-N=N-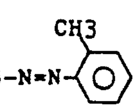 | 205-206 |

| | | | | | |
|---|---|---|---|---|---|
| 146 | S | O | 2,6-F2 | 2-CH3 4-N=N-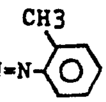 | 177-178 |
| 147 | " | " | H | 2-CF3 | 145-147 |
| 148 | " | " | " | 3-CF3 | 135-138 |
| 149 | " | " | 2-Cl | 2-Br 4-OCF3 | 194-195 |
| 150 | " | " | 2-CH3 | " | 121-122 |
| 151 | " | " | 2,6-F2 | " | 134-135 |
| 152 | " | " | 2-Cl | 2-Br 4-F | 178-179 |
| 153 | " | " | 2,6-F2 | " | 194-197 |
| 154 | " | " | 2-Cl | 2,6-F2 4-Br | 212-216 |
| 155 | " | " | 2,6-F2 | " | 181-185 dec. |
| 156 | " | " | 2-Cl | 2,6-F2 | 159-165 |
| 157 | " | " | 2,6-F2 | " | 221-223 |
| 158 | " | " | 2-Cl | 2,6-Cl2 4-OCF2Cl | 158-160 |
| 159 | " | " | 2,6-F2 | " | 116-117 |
| 160 | " | " | 2,6-Cl2 | 2,6-Cl2 | 250-253 |
| 161 | " | " | H | 2,6-(CH3)2 | 196-197 |
| 162 | " | " | 2-Cl | 2,5-F2 4-Cl | 224-228 |
| 163 | " | " | " | 2,5-F2 4-Br | 232-235 |
| 164 | " | " | 2,6-F2 | " | 207-209 |
| 165 | " | " | 2-Cl | 2,5-Cl2 4-CF3 | 194-195 |
| 166 | " | " | 2,6-F2 | " | 175-177 |
| 167 | " | " | 2-F | 2,5-Cl2 3-CF3 | 182-183 |
| 168 | " | " | 2-Cl | " | 211-214 |
| 169 | " | " | 2,6-F2 | " | 167-175 |
| 170 | " | " | 2,5-F2 | " | 181-185 |
| 171 | " | " | 2,4-F2 | " | 181-185 |
| 172 | " | " | 2-Cl 6-F | " | 104-106 |
| 173 | " | " | 2-CH3 | " | 161-163 |

| | | | | | |
|---|---|---|---|---|---|
| 174 | S | O | 2-Br | 2,5-Cl2 3-CF3 | 195-198 |
| 175 | " | " | 2-Cl | 2,4-F2 5-Cl | 248-249 |
| 176 | " | " | 2,6-F2 | " | 226-228 |
| 177 | " | " | 2-Cl | 2,4-F2 3-Cl | 258-261 |
| 178 | " | " | 2,6-F2 | " | 226 |
| 179 | " | " | 2-Cl | 2,4-F2 3,5-Cl2 | 255-257 |
| 180 | " | " | 2,6-F2 | " | 213-215 |
| 181 | " | " | 2-Cl | 2,4-Cl2 5-OCH3 | 173-174 |
| 182 | " | " | 2,6-F2 | " | 242-244 |
| 183 | " | " | 2-Cl | 2,4-Cl2 5-OCF3 | 186-189 |
| 184 | " | " | 2,6-F2 | " | 158-160 |
| 185 | " | " | 2-Cl | 2,4-Cl2 5-OCF2CF2H | 161-163 |
| 186 | " | " | 2,6-F2 | " | 125-126 |
| 187 | " | " | 2-Cl | 2,4-Cl2 5-CF3 | 203-210 |
| 188 | " | " | 2,6-F2 | " | 175-178 |
| 189 | " | " | 2-Cl | 2,4-Cl2 3-CF3 | 219-221 |
| 190 | " | " | 2,6-F2 | " | 220-224 |
| 191 | " | " | H | 2,4-Cl2 | 196-198 |
| 192 | " | " | 2-Cl | 2,4,5-F3 | 227-229 |
| 193 | " | " | 2,6-F2 | " | 204-206 |
| 194 | " | " | 2-Cl | 2,3-Cl2 5-OCF3 | 186-189 |
| 195 | " | " | 2,6-F2 | " | 182-184 |
| 196 | " | " | 2-Cl | 2,3-Cl2 5-CF3 | 167-170 |
| 197 | " | " | 2-F | 2,3-Cl2 4-OCF3 | 196-198 |
| 198 | " | " | 2-Cl | " | 176-178 |
| 199 | " | " | 2,6-F2 | " | 184-186 |
| 200 | " | " | " | 2,3,5-Cl3 4-OCF2CF2H | 157-159 |
| 201 | " | " | " | 2,3,5-Cl3 | 211-212 |
| 202 | " | " | 2-Cl | 2,3,4-Cl3 | 250 |
| 203 | " | " | 2,6-F2 | " | 253-255 |

| | | | | | |
|---|---|---|---|---|---|
| 204 | S | O | 2,6-F2 | 2,3,4,5-Cl4 | 203-205 |
| 205 | " | " | " | 2,3,4,5,6-F5 | 167-168 |
| 206 | " | " | 2-F | 2,3,5-Cl3 4-OCF2CF2H | 174-176 |
| 207 | " | " | 2,6-F2 | 2,5-F2 3-CF3 | 140-142 |
| 208 | " | " | 2-F | " | 145 |
| 209 | " | " | 2,6-F2 | 2,5-Cl2 4-OCF3 | 174-177 |
| 210 | " | " | 2-F | " | 181-183 |
| 211 | " | " | 2-Cl | " | 178-179 |
| 212 | " | " | 2,6-F2 | 2-CH3 4-OCF3 | 148-149 |
| 213 | " | " | 2-F | " | 177-178 |
| 214 | " | S | 2-Cl | 4-OCF3 | 75-77 |
| 215 | " | O | 2,6-F2 | 2-Cl 3-CF3 5-F | 156-158 |
| 216 | " | " | 2-F | " | 174-175 |
| 217 | " | " | 2-Cl | " | 168-172 |
| 218 | " | " | 2-F | 2-Cl 4-OCF3 | 174-175 |
| 219 | " | " | " | 3-F 4-OCF3 | 143-146 |
| 220 | " | " | 2,6-F2 | 4-SCH3 | 179-180 |
| 221 | " | " | 2-Cl | 3-F 4-OCF3 | 142-144 |
| 222 | " | " | 2-F | 2-Br 4-OCF3 | 176-178 |
| 223 | " | " | 2,6-F2 | 2-F 3-CF3 4-Cl | 185-187 |
| 224 | " | " | 2-F | " | 173-175 |
| 225 | " | " | 2-CH3 | 3-F 4-OCF3 | 91-94 |
| 226 | " | " | 2-F | 2,3-F2 4-OCF3 | 132-134 |
| 227 | " | " | " | 2-OCF3 3,4-F2 | 153-156 |
| 228 | " | " | 2,6-F2 | 2,3-F2 4-OCF3 | 142-145 |
| 229 | " | " | 2-F | 3,5-Cl2 4-OCF2CF2H | 133-134 |
| 230 | " | " | 2,6-F2 | 3-CH3 4-OCF3 | 128-130 |
| 231 | " | " | 2-F | " | 127-130 |
| 232 | " | " | 2,6-F2 | 2,4-F2 3-CF3 | 153-155 |
| 233 | " | " | 2-F | " | 143-145 |

| | | | | | |
|---|---|---|---|---|---|
| 234 | S | O | 2-OCH3 | 3-Cl 4-OCF3 | 178-179.5 |
| 235 | " | " | 2-F | 3-CH3 4-OCF2CF2H | 130-131 |
| 236 | O | " | 2-Cl | 2,5-Cl2 3-CF3 | 155-158 |
| 237 | " | " | 2-F | 2,5-Cl2 3-CF3 | 163-165 |
| 238 | " | " | 2,6-F2 | 3-Cl 4-CF3 | 160-162 |
| 239 | " | " | 2-CH3 | " | 149-150 |
| 240 | " | " | 2-Cl | " | 165-166 |
| 241 | " | " | 2-Cl 6-F | " | 138-141 |
| 242 | " | " | 2-Br | " | 176-179 |
| 243 | " | " | 2-CF3 | " | 159-162 |
| 244 | " | " | 2,6-F2 | 2,5-Cl2 3-CF3 | 143-145 |
| 245 | " | " | " | 2-F 3-CF3 5-Cl | 127-128 |
| 246 | " | " | 2-CF3 | " | 80-81 |
| 247 | " | " | 2,6-F2 | 3-Cl 4-OCF3 | 121-122 |
| 248 | " | " | 2-CH3 | " | 128 |
| 249 | " | " | 2-Cl | 4-CF3 | 150-153 |
| 250 | " | " | 2,6-F2 | " | 143-145 |
| 251 | " | " | 2-CH3 | 4-OCF3 | 116-120 |
| 252 | " | " | 2,6-F2 | " | 91.5-93.5 |
| 253 | " | " | 2-Cl | 4-OCF2CFClH | 127-129 |
| 254 | " | " | 2,6-F2 | " | 97-102 |
| 255 | " | " | 2-Cl | 4-OCF3 | 129-132 |
| 256 | " | " | 2,6-F2 | 4-Cl | 178-181 |
| 257 | " | " | H | " | 177-178 |
| 258 | " | " | 2,6-F2 | 3-CF3 4-Cl | 186-187 |
| 259 | " | " | 2,6-Cl2 | 4-Cl | 112-115 |
| 260 | " | " | 4-CH3 | " | 178-181 |
| 261 | " | " | 2,6-F2 | 2,5-F2 3-CF3 | 126.5-128 |
| 262 | " | " | " | 2-Cl 3-CF3 5-F | 149-150 |
| 263 | " | " | 2-CH3 | 2,5-Cl2 3-CF3 | 110-112 |

| | | | | | |
|---|---|---|---|---|---|
| 264 | O | O | 2,4-F2 | 2,5-Cl2 3-CF3 | 168-170 |
| 265 | " | " | 2-Cl 6-F | " | 91-93 |
| 266 | " | " | 2,6-F2 | 3-OC2H5 4-CF3 | 107-109 |
| 267 | " | " | 2,6-F2 | 4-O- 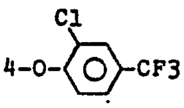 | 66-67 |
| 268 | " | " | " | 3-CH3 4-OCF2CF2H | 132-133 |
| 269 | " | " | " | 3-O- 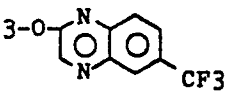 | 146-147 |
| 270 | S | S | " | 3-Cl 4-CF3 | 141-143 |
| 271 | " | O | " | 2,4-F2 3,5-Cl2 | 213-215 |
| 272 | " | " | 2-Cl | " | 255-257 |

As already mentioned, the compound having the formula (I) exhibits an outstanding pesticidal efficacy and a pesticidal composition containing the compound as an active ingredient may be formulated by mixing suitable carrieres in a form generally used in agricultural pesticide, such as wettable powder, emulsifiable concentrate, water-soluble powder, dust, granular formulation, suspension concentrate or the like.

As solid carriers, cereal flours such as soy bean flour and wheat flour, ground minerals such as diatomaceous earth, apatite, gypsum, talc, bentonite and clay, and organic or inorganic compounds such as sodium benzoate, urea and sodium sulfate may be used.

As liquid carriers, vegetable oil, mineral oil, petroleum such as kerosine, solvent naphtha and xylene, cyclohexane, cyclohexanone, dimethylformamide, dimethylsulfoxide, methylisobutyl ketone and water may be used.

A surfactant may, if necessary, be added in order to give a homogeneous and stable formulation.

The concentration of the active ingredient in a pesticidal composition may vary according to type of formulation, and is, for example, in a range of 5 - 70 weight percent, preferably 10 - 30 weight percent, in wettable powder; 5 - 30 weight percent, preferably 10 - 20 weight percent, in emulsifiable concentrate; 5 - 80 weight percent, preferably 30 - 60 weight percent, in water-soluble powder; 1 - 10 weight percent, preferably 2 - 5 weight percent, in dust; 5 - 40 weight percent, preferably 10 - 30 weight percent, in suspension concentrate; 1 - 10 weight percent, preferably 2 - 5 weight percent, in granular formulation.

The wettable powder, the emulsifiable concentrate, water-soluble powder and suspension concentrate are diluted with water to the specified concentrations and used as a liquid suspension or a liquid emulsion to spray

over the plants.

The dust and granular formulation are directly used for spraying over the plants.

Examples of the pesticidal composition of this invention are as mentioned below, but the scope of the invention shall not be limitted to those:

Example 6 : Emulsifiable Concentrate

| | | |
|---|---|---|
| Compound of this invention | 10 | parts by weight |
| Calcium dodecylbenzenesulfonate | 5 | " |
| Dimethylformamide | 40 | " |
| Xylene | 40 | " |
| Polyoxyethylene styrylphenyl ether | 3 | " |
| Polyoxyethylene alkylaryl ether | 2 | " |

Those are mixed together to provide an emulsifiable concentrate containing 10% of active ingredient. At use, it is diluted with water to the desired concentration of the emulsion.

Example 7 : Wettable Powder

| | | |
|---|---|---|
| Compound of this invention | 20 | parts by weight |
| Talc | 75 | " |
| Sodium lignin sulfonate | 3 | " |
| Sodium stearate | 2 | " |

Those are mixed together to provide a wettable powder containing 20% of active ingredient. At use, it is diluted with water to the desired concentration of the suspension.

Example 8 : Water Soluble Powder

| | | |
|---|---|---|
| Compound of this invention | 50 | parts by weight |

| | |
|---|---|
| Sodium alkylsulfosuccinate | 10 parts by weight |
| Sodium benzoate | 40 " |

Those are mixed to provide a water-soluble powder containing 50% of active ingredient. At use, it is diluted with water to obtain a solution or a suspension at the desired concentration.

Example 9 : Dust

| | |
|---|---|
| Compound of this invention | 5 parts by weight |
| Talc | 92 " |
| Silica | 3 " |

Those are mixed together to provide a dust containing 5% of active ingredient.

Further, it goes without saying that the compound indicates a sufficient insecticidal efficacy, but, in the pesticidal composition, one kind or two kinds or more of other insecticidal compounds may be mixed in order to give a rapid insecticidal action or extend its spectrum (hereinafter called as "mixed composition"), because the compound of this invention indicates a slow-acting effect on larval or lacks the sufficient insecticidal effect against adult insect.

In the mixed composition, the compound can be used with one kind or two kinds or more of fungicidal and/or acaricidal compound(s) as well as the insecticidal compound having the rapid action.

Typical examples of insecticidal compounds usable together with the compound of this invention in the mixed composition as active ingredient(s) are set forth below:

(Organophosphorous compounds or carbamates)

fenthion, fenitrotion, diazion, chlorpyrifos, ESP, vamidothion,

phenthoate, dimethoate, formotion, malathion, trichlorfon, thiomethon, dichlorvos, acephate, cyanophos, pyrimiphos methyl, isoxathin, pyridaphenthion, DMTP, prothiophos, sulprofos, profenofos, CVMP, salithion, EPN, CYP, aldicarb, propoxur, pyrimicarb, methomyl, cartap, carbaryl, thiodicarb, carbosulfen, carbosulfan, nicotine.

(Pyrethroids)

permethrin, cypermethrin, decamethrin, fenvalerate, fenpropathrin, cyhalothrin, flucythrate, fencyclate, tetramethrin, cyfluthrin, fluvalinate, pyrethrin, allethrin, tetramethrin, resmethrin, barthrin, dimethrin, propathrin, prothrin.

The pesticidal activity of the compound was evaluated by the following tests.

Test 1 : Insecticidal activity against Armyworm

An emulsifiable concentrate formulated according to the aforesaid Example 6 was diluted with water to the concentrations of 500 ppm and 125 ppm of the compound. A leaf of corn was immersed in the emulsion formulation for 30 seconds and air-dried. The treated leaf was put in a petri dish enclosing 5 of third instar larvae of the armyworm, and the petri dish was capped with a sheet of glass. The petri dish was placed in a room kept at 25°C and 65% relative humidity, and the mortality was investigated after 120 hours. The results obtained from replicated tests in two times are shown in Table 2.

Table 2

| Compound No. | Mortality after 120 hours | |
|---|---|---|
| | 500 ppm | 125 ppm |
| 1 | 100 | 100 |
| 2 | 100 | 100 |
| 3 | 100 | 100 |
| 4 | 100 | 100 |
| 5 | 100 | 100 |
| 6 | 100 | 100 |
| 7 | 100 | 100 |
| 8 | 100 | 100 |
| 9 | 100 | 100 |
| 11 | 100 | 100 |
| 12 | 100 | 100 |
| 13 | 100 | 100 |
| 14 | 100 | 100 |
| 15 | 100 | 100 |
| 16 | 100 | 100 |
| 17 | 100 | 100 |
| 18 | 100 | 100 |
| 19 | 100 | 100 |
| 20 | 100 | 100 |
| 21 | 100 | 100 |
| 26 | 100 | 100 |
| 27 | 100 | 100 |
| 28 | 100 | 100 |
| 29 | 100 | 100 |
| 30 | 100 | 100 |
| 33 | 100 | 100 |

| | | |
|---|---|---|
| 34 | 100 | 100 |
| 35 | 100 | 100 |
| 36 | 100 | 100 |
| 37 | 100 | 100 |
| 38 | 100 | 100 |
| 42 | 100 | 100 |
| 45 | 100 | 100 |
| 46 | 100 | 100 |
| 47 | 100 | 100 |
| 48 | 100 | 100 |
| 49 | 100 | 100 |
| 50 | 100 | 100 |
| 51 | 100 | 100 |
| 53 | 100 | 100 |
| 54 | 100 | 100 |
| 55 | 100 | 100 |
| 56 | 100 | 100 |
| 57 | 100 | 100 |
| 58 | 100 | 100 |
| 59 | 100 | 100 |
| 60 | 100 | 100 |
| 61 | 100 | 100 |
| 62 | 100 | 100 |
| 63 | 100 | 100 |
| 64 | 100 | 100 |
| 65 | 100 | 100 |
| 66 | 100 | 100 |
| 67 | 100 | 100 |
| 68 | 100 | 100 |
| 69 | 100 | 100 |

| | | |
|---|---|---|
| 70 | 100 | 100 |
| 71 | 100 | 100 |
| 72 | 100 | 100 |
| 73 | 100 | 100 |
| 76 | 100 | 100 |
| 77 | 100 | 100 |
| 78 | 100 | 100 |
| 79 | 100 | 100 |
| 80 | 100 | 100 |
| 81 | 100 | 100 |
| 82 | 100 | 100 |
| 83 | 100 | 100 |
| 84 | 100 | 100 |
| 85 | 100 | 100 |
| 86 | 100 | 100 |
| 87 | 100 | 100 |
| 97 | 100 | 100 |
| 98 | 100 | 100 |
| 99 | 100 | 100 |
| 100 | 100 | 100 |
| 101 | 100 | 100 |
| 102 | 100 | 100 |
| 103 | 100 | 100 |
| 104 | 100 | 100 |
| 105 | 100 | 100 |
| 106 | 100 | 100 |
| 107 | 100 | 100 |
| 111 | 100 | 100 |
| 112 | 100 | 100 |
| 113 | 100 | 100 |

| | | |
|---|---|---|
| 115 | 100 | 100 |
| 116 | 100 | 100 |
| 117 | 100 | 100 |
| 123 | 100 | 100 |
| 124 | 100 | 100 |
| 125 | 100 | 100 |
| 127 | 100 | 100 |
| 128 | 100 | 100 |
| 129 | 100 | 100 |
| 131 | 100 | 100 |
| 133 | 100 | 100 |
| 135 | 100 | 100 |
| 140 | 100 | 100 |
| 141 | 100 | 100 |
| 142 | 100 | 100 |
| 143 | 100 | 100 |
| 144 | 100 | 100 |
| 149 | 100 | 100 |
| 151 | 100 | 100 |
| 155 | 100 | 100 |
| 164 | 100 | 100 |
| 165 | 100 | 100 |
| 166 | 100 | 100 |
| 167 | 100 | 100 |
| 168 | 100 | 100 |
| 169 | 100 | 100 |
| 170 | 100 | 100 |
| 171 | 100 | 100 |
| 172 | 100 | 100 |
| 173 | 100 | 100 |

| | | |
|---|---|---|
| 174 | 100 | 100 |
| 177 | 100 | 100 |
| 180 | 100 | 100 |
| 189 | 100 | 100 |
| 190 | 100 | 100 |
| 194 | 100 | 100 |
| 195 | 100 | 100 |
| 196 | 100 | 100 |
| 197 | 100 | 100 |
| 198 | 100 | 100 |
| 201 | 100 | 100 |
| 202 | 100 | 100 |
| 206 | 100 | 100 |
| 207 | 100 | 100 |
| 208 | 100 | 100 |
| 209 | 100 | 100 |
| 210 | 100 | 100 |
| 211 | 100 | 100 |
| 212 | 100 | 100 |
| 213 | 100 | 100 |
| 214 | 100 | 100 |
| 215 | 100 | 100 |
| 216 | 100 | 100 |
| 217 | 100 | 100 |
| 218 | 100 | 100 |
| 219 | 100 | 100 |
| 221 | 100 | 100 |
| 223 | 100 | 100 |
| 224 | 100 | 100 |
| 225 | 100 | 100 |

| | | |
|---|---|---|
| 226 | 100 | 100 |
| 227 | 100 | 100 |
| 228 | 100 | 100 |
| 229 | 100 | 100 |
| 230 | 100 | 100 |
| 231 | 100 | 100 |
| 236 | 100 | 100 |
| 237 | 100 | 100 |
| 238 | 100 | 100 |
| 239 | 100 | 100 |
| 240 | 100 | 100 |
| 241 | 100 | 100 |
| 242 | 100 | 100 |
| 243 | 100 | 100 |
| 244 | 100 | 100 |
| 245 | 100 | 100 |
| 246 | 100 | 100 |
| 247 | 100 | 100 |
| 248 | 100 | 100 |
| 249 | 100 | 100 |
| 250 | 100 | 100 |
| 251 | 100 | 100 |
| 252 | 100 | 100 |
| 253 | 100 | 100 |
| 254 | 100 | 100 |
| 255 | 100 | 100 |
| 256 | 100 | 100 |
| 258 | 100 | 100 |
| 259 | 100 | 100 |
| 261 | 100 | 100 |

| | | |
|---|---|---|
| 262 | 100 | 100 |
| 263 | 100 | 100 |
| 264 | 100 | 100 |
| 265 | 100 | 100 |
| Comparative Compound 1* | 100 | 100 |
| " 2* | 0 | 0 |

* Comparative Compound 1 : 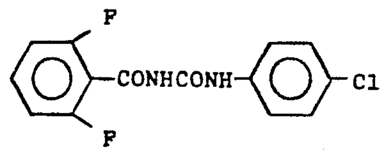 (diflubenzuron)

" " 2 : 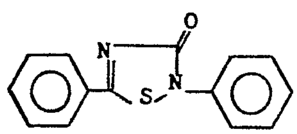

Test 2 : Insecticidal activity against Tobacco cutworm

A wettable powder formulated according to the aforesaid Example 7 was diluted with water to the concentrations of 125 ppm and 31.3 ppm of the compound.

A leaf of sweet potato was immersed in the suspension formulation for 30 seconds and air-dried.

The treated leaf was put in a petri dish of 9 cm diameter in which 5 of third instar larvae of tobacco cutworm were released and the petri dish was capped with a sheet of glass. The petri dish was placed in a room at 25°C and 65% relative humidity, and the mortality was investigated after 120 hours. The results obtained from replicated tests in two times are shown in Table 3.

Table 3

| Compound No. | Mortality after 120 hours | |
|---|---|---|
| | 125 ppm | 31.3 ppm |
| 1 | 100 | 100 |
| 3 | 100 | 100 |
| 4 | 100 | 100 |
| 6 | 100 | 100 |
| 7 | 100 | 100 |
| 8 | 100 | 100 |
| 9 | 100 | 100 |
| 11 | 100 | 100 |
| 12 | 100 | 100 |
| 13 | 100 | 100 |
| 14 | 100 | 100 |
| 15 | 100 | 100 |
| 16 | 100 | 100 |
| 17 | 100 | 100 |
| 18 | 100 | 100 |
| 20 | 100 | 100 |
| 28 | 100 | 100 |
| 29 | 100 | 100 |
| 30 | 100 | 100 |
| 34 | 100 | 100 |
| 37 | 100 | 100 |
| 40 | 100 | 100 |
| 41 | 100 | 100 |
| 42 | 100 | 100 |
| 49 | 100 | 100 |
| 50 | 100 | 100 |

| | | |
|---|---|---|
| 51 | 100 | 100 |
| 54 | 100 | 100 |
| 55 | 100 | 100 |
| 56 | 100 | 100 |
| 57 | 100 | 100 |
| 58 | 100 | 100 |
| 59 | 100 | 100 |
| 60 | 100 | 100 |
| 61 | 100 | 100 |
| 63 | 100 | 100 |
| 64 | 100 | 100 |
| 65 | 100 | 100 |
| 66 | 100 | 100 |
| 68 | 100 | 100 |
| 69 | 100 | 100 |
| 70 | 100 | 100 |
| 71 | 100 | 100 |
| 72 | 100 | 100 |
| 73 | 100 | 100 |
| 74 | 100 | 100 |
| 75 | 100 | 100 |
| 77 | 100 | 100 |
| 78 | 100 | 100 |
| 79 | 100 | 100 |
| 80 | 100 | 100 |
| 86 | 100 | 100 |
| 87 | 100 | 100 |
| 88 | 100 | 100 |
| 89 | 100 | 100 |
| 96 | 100 | 80 |

| | | |
|---|---|---|
| 97 | 100 | 100 |
| 98 | 100 | 100 |
| 99 | 100 | 100 |
| 100 | 100 | 100 |
| 101 | 100 | 100 |
| 102 | 100 | 100 |
| 103 | 100 | 100 |
| 106 | 100 | 100 |
| 107 | 100 | 100 |
| 111 | 100 | 100 |
| 112 | 100 | 100 |
| 113 | 100 | 100 |
| 115 | 100 | 100 |
| 117 | 100 | 100 |
| 124 | 100 | 100 |
| 127 | 100 | 100 |
| 128 | 100 | 100 |
| 129 | 100 | 100 |
| 131 | 100 | 100 |
| 134 | 100 | 100 |
| 135 | 100 | 100 |
| 137 | 100 | 100 |
| 141 | 100 | 100 |
| 142 | 100 | 100 |
| 143 | 100 | 100 |
| 144 | 100 | 100 |
| 146 | 100 | 100 |
| 149 | 100 | 100 |
| 151 | 100 | 100 |
| 153 | 100 | 100 |

| | | |
|---|---|---|
| 159 | 100 | 100 |
| 165 | 100 | 100 |
| 166 | 100 | 100 |
| 167 | 100 | 100 |
| 168 | 100 | 100 |
| 169 | 100 | 100 |
| 171 | 100 | 100 |
| 172 | 100 | 100 |
| 173 | 100 | 100 |
| 174 | 100 | 100 |
| 180 | 100 | 100 |
| 194 | 100 | 100 |
| 195 | 100 | 100 |
| 196 | 100 | 100 |
| 197 | 100 | 100 |
| 198 | 100 | 100 |
| 199 | 100 | 100 |
| 200 | 100 | 100 |
| 201 | 100 | 100 |
| 206 | 100 | 100 |
| 207 | 100 | 100 |
| 208 | 100 | 100 |
| 209 | 100 | 100 |
| 210 | 100 | 100 |
| 211 | 100 | 100 |
| 212 | 100 | 100 |
| 213 | 100 | 100 |
| 214 | 100 | 100 |
| 215 | 100 | 100 |
| 216 | 100 | 100 |

| | | |
|---|---|---|
| 217 | 100 | 100 |
| 218 | 100 | 100 |
| 219 | 100 | 100 |
| 221 | 100 | 100 |
| 223 | 100 | 100 |
| 224 | 100 | 100 |
| 225 | 100 | 100 |
| 226 | 100 | 100 |
| 228 | 100 | 100 |
| 229 | 100 | 100 |
| 230 | 100 | 100 |
| 231 | 100 | 100 |
| 236 | 100 | 100 |
| 237 | 100 | 100 |
| 238 | 100 | 100 |
| 239 | 100 | 100 |
| 240 | 100 | 100 |
| 241 | 100 | 100 |
| 242 | 100 | 100 |
| 244 | 100 | 100 |
| 245 | 100 | 100 |
| 246 | 100 | 100 |
| 247 | 100 | 100 |
| 254 | 100 | 100 |
| 258 | 100 | 100 |
| 261 | 100 | 100 |
| 262 | 100 | 100 |
| 263 | 100 | 100 |
| 264 | 100 | 100 |
| 265 | 100 | 100 |

| | | |
|---|---|---|
| Comparative Compound 1* | 80 | 10 |

* Comparative Compound 1 : 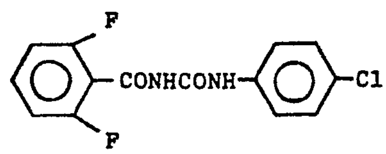 (diflubenzuron)

Test 3 : Insecticidal activity against Diamondback moth

An emulsifiable concentrate was diluted with water to the concentrations of 500 ppm and 125 ppm of the compound.

A leaf of cabbage was immersed in the emulsion formulation for 30 seconds and air-dried.

The treated leaf was put in a petri dish of 9 cm diameter in which 5 of third instar larvae of diamondback moth were released, and the petri dish was capped with a sheet of glass. The petri dish was kept in a room at 25°C and 65% relative humidity, and the mortality was investigated after 120 hours. The results obtained from replicated test in two times are shown in Table 4.

Table 4

| Compound No. | Mortality after 120 hours | |
|---|---|---|
| | 500 ppm | 125 ppm |
| 6 | 100 | 100 |
| 7 | 100 | 100 |
| 8 | 100 | 100 |
| 11 | 100 | 100 |
| 12 | 100 | 100 |
| 13 | 100 | 100 |
| 17 | 100 | 100 |
| 32 | 100 | 100 |
| 33 | 100 | 100 |
| 55 | 100 | 100 |
| 57 | 100 | 100 |
| 60 | 100 | 100 |
| 68 | 100 | 100 |
| 75 | 100 | 100 |
| 77 | 100 | 100 |
| 80 | 100 | 100 |
| 87 | 100 | 100 |
| 97 | 100 | 100 |
| 98 | 100 | 100 |
| 102 | 100 | 100 |
| 106 | 100 | 100 |
| 107 | 100 | 100 |
| 117 | 100 | 100 |
| 140 | 100 | 100 |
| 163 | 100 | 100 |
| 164 | 100 | 100 |

| | | |
|---|---|---|
| 167 | 100 | 100 |
| 188 | 100 | 100 |
| 189 | 100 | 100 |
| 190 | 100 | 100 |
| Comparative Compound 1* | 60 | 0 |

* Comparative Compound 1 : 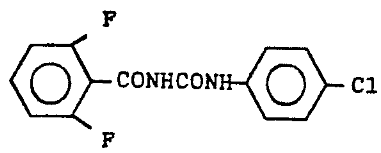 (diflubenzuron)

<u>Test 4</u> : Ovicidal activity against eggs of Tobacco cutworm

An emulsifiable concentrate was diluted with water to the concentrations of 500 ppm and 125 ppm of the compound.

Eggs of tobacco cutworm were immersed in the emulsion formulation for 30 seconds and air-dried. The eggs were put in a petri dish and the petri dish was capped with a sheet of glass. The petri dish was kept in a room at 25°C and 65% relative humidity, and the ovicidal activity was investigated after 7 days. The results are shown in Table 5.

Table 5

| Compound No. | Ovicidal Activity after 7 days | |
|---|---|---|
| | 500 ppm | 125 ppm |
| 1 | 100 | 100 |
| 2 | 100 | 100 |
| 6 | 100 | 100 |
| 7 | 100 | 100 |
| 8 | 100 | 100 |
| 9 | 100 | 100 |
| 11 | 100 | 100 |
| 12 | 100 | 100 |
| 16 | 100 | 100 |
| 17 | 100 | 100 |
| 18 | 100 | 100 |
| 26 | 100 | 100 |
| 33 | 100 | 100 |
| 37 | 100 | 100 |
| 38 | 100 | 100 |
| 50 | 100 | 100 |
| 54 | 100 | 100 |
| 56 | 100 | 100 |
| 57 | 100 | 100 |
| 58 | 100 | 100 |
| 60 | 100 | 100 |
| 61 | 100 | 90 |
| 63 | 100 | 100 |
| 68 | 100 | 100 |
| 77 | 100 | 100 |
| 97 | 100 | 100 |

| | | |
|---|---|---|
| 100 | 100 | 100 |
| 111 | 100 | 100 |
| 167 | 100 | 100 |
| 197 | 100 | 100 |
| 208 | 100 | 100 |
| 210 | 100 | 100 |
| 212 | 100 | 100 |
| 213 | 100 | 100 |
| 214 | 100 | 100 |
| 215 | 100 | 100 |
| 216 | 100 | 100 |
| 219 | 100 | 100 |
| 221 | 100 | 100 |
| 225 | 100 | 100 |
| 226 | 100 | 100 |
| 227 | 100 | 100 |
| 228 | 100 | 100 |
| 229 | 100 | 100 |
| 230 | 100 | 100 |
| 231 | 100 | 100 |
| 251 | 100 | 100 |
| 252 | 100 | 100 |
| Comparative Compound 1* | 100 | 100 |

* Comparative Compound 1 : 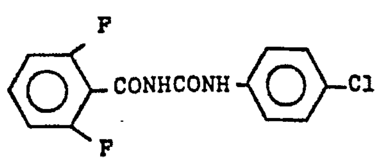 (diflubenzuron)

Test 5 : Downy mildew (Pseudoperonospora cubensis) of cucumber

An aqueous solution having a specified concentration which was prepared from the wettable powder of each test compound was sprayed over the young seedling of cucumber (Variety: Sagami hanjiro) cultiivated for about 3 weeks.

The seedling was air-dried and inoculated with a liquid suspension containing the zoosporangium of Pseudoperonospora cubensis collected from the cucumber leaf affected by downy mildew by means of spraying.

The seedling was placed in the inoculating box having 100% of relative humidity at 25°C.

On the second day after the inoculation, the plant was transferred to the room at a temperature of 23 - 28°C.

On the 7th day after the inoculation, the disease occurrence degree of each cucumber leaf was investigated in compliance with the following criteria:

Investigating criteria:

| Disease index | 0 | 0.5 | 1 | 2 | 3 | 4 |
|---|---|---|---|---|---|---|
| Surface area of leaf affected with disease lesion | healthy | status of disease lesion is fine | 25% or less | 50% or less | 75% or less | 75% or more |

The preventive value in the treated plot was calculated by the following calculating formula:

$$\text{Preventive Value } (\%) = \left( 1 - \frac{\text{Average disease index in treated plot}}{\text{Average disease index in untreated plot}} \right) \times 100$$

The results are shown in Table 6 as follows:

Table 6

| Compound No. | Concentration of Active ingredient (ppm) | Preventive Value (%) |
|---|---|---|
| 2 | 400 | 87 |
| 8 | 400 | 100 |
| 9 | 400 | 87 |
| 11 | 400 | 87 |
| 13 | 400 | 81 |
| 16 | 400 | 87 |
| 18 | 400 | 94 |
| 26 | 400 | 81 |
| 32 | 400 | 94 |
| 33 | 400 | 94 |
| 35 | 400 | 81 |
| 36 | 400 | 94 |
| 38 | 400 | 100 |
| 40 | 400 | 81 |
| 42 | 400 | 81 |
| 43 | 400 | 94 |
| 44 | 400 | 87 |
| 45 | 400 | 94 |
| 50 | 400 | 100 |
| 51 | 400 | 100 |
| 57 | 400 | 87 |
| 59 | 400 | 81 |
| 64 | 400 | 100 |
| 68 | 400 | 87 |
| 74 | 400 | 81 |
| 77 | 400 | 87 |

| | | |
|---|---|---|
| 80 | 400 | 100 |
| 96 | 400 | 94 |
| 110 | 400 | 87 |
| 119 | 400 | 87 |
| 122 | 400 | 94 |
| 123 | 400 | 87 |
| 128 | 400 | 94 |
| 134 | 400 | 81 |
| 135 | 400 | 81 |
| 138 | 400 | 100 |
| 139 | 400 | 100 |
| 144 | 400 | 100 |
| 150 | 400 | 87 |
| 152 | 400 | 94 |
| 164 | 400 | 100 |
| 166 | 400 | 87 |
| 169 | 400 | 87 |
| 170 | 400 | 81 |
| 172 | 400 | 100 |
| 173 | 400 | 81 |
| 178 | 400 | 87 |
| 184 | 400 | 100 |
| 186 | 400 | 94 |
| 193 | 400 | 87 |
| 195 | 400 | 87 |
| 199 | 400 | 81 |
| 212 | 400 | 87 |
| 213 | 400 | 94 |
| 218 | 400 | 100 |
| 220 | 400 | 81 |

| | | |
|---|---|---|
| 223 | 400 | 94 |
| 224 | 400 | 100 |
| 225 | 400 | 94 |
| 226 | 400 | 87 |
| 228 | 400 | 81 |
| 230 | 400 | 100 |
| 231 | 400 | 87 |
| 244 | 400 | 81 |
| 245 | 400 | 81 |
| Comparative Compound 3** | 400 | 81 |
| " 4** | 400 | 80 |

** Comparative Compound 3 : tetrachloroisophthalonitrile 75% w.p.

" " 4 : manganese ethylenebisdithiocarbamate 75% w.p.

What we claim is:

1. A compound having the formula:

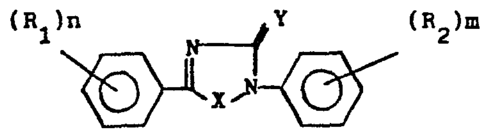

wherein each of X and Y is oxygen or sulfur;

$R_1$ is same or different substituent(s) selected from the group consisting of halogen, $C_{1-3}$ alkyl, $C_{1-3}$ haloalkyl and $C_{1-3}$ alkoxy;

$R_2$ is same or different substituent selected from the group consisting of halogen, saturated or unsaturated $C_{1-6}$ hydrocarbon (which may be substituted by halogen), $C_{1-6}$ alkoxycarbonyl, $C_{1-6}$ alkyl substituted amino, phenylazo (which may be substituted by $C_{1-6}$ alkyl) and -A-R wherein

A is oxygen or sulfur, and

R is saturated or unsaturated $C_{1-6}$ hydrocarbon (which may be substituted by halogen, phenyl and/or phenoxy), or aryl containing not more than ten members and two or less thereof may be nitrogen (which may be substituted by halogen, cyano, $C_{1-6}$ haloalkyl and/or nitro);

n is zero or an integer of 1 to 3; and

m is an integer of 1 to 5.

2. A compound according to claim 1, wherein X is sulfur and Y is oxygen.

3. A compound according to claim 1, wherein X and Y are oxygen.

4. An insecticidal composition comprising an inert carrier and an effective amount of a compound of claim 1.

5. An insecticidal composition comprising an inert carrier and an effective amount of a compound of claim 2.

6. An insecticidal composition comprising an inert carrier and an effective amount of a compound of claim 3.

7. A process for the controlling insects comprising applying to the locus of plants an effective amount of a compound of claim 1.

8. A process for the controlling insects comprising applying to the locus of plants an effective amount of a compound of claim 2.

9. A process for the controlling insects comprising applying to the locus of plants an effective amount of a compound of claim 3.

10. A fungicidal composition comprising an inert carrier and an effective amount of a compound of claim 1.

11. A process for the controlling fungi comprising applying to the locus of the plants an effective amount of a compound of claim 1.

12. A process for the preparation of a compound having the formula:

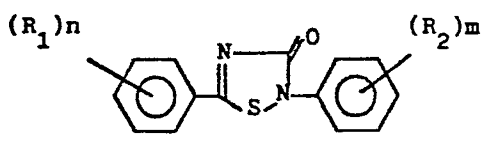

which comprises reacting a compound having the formula:

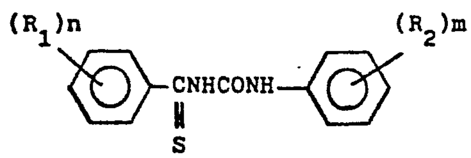

with an oxdizing agent

wherein $R_1$ is same or different substituent(s) selected from the group consisting of halogen, $C_{1-3}$ alkyl, $C_{1-3}$ haloalkyl and $C_{1-3}$ alkoxy;

$R_2$ is same or different substituent selected from the group consisting of halogen, saturated or unsaturated $C_{1-6}$ hydrocarbon (which may be substituted by halogen), $C_{1-6}$ alkoxycarbonyl, $C_{1-6}$ alkyl substituted amino, phenylazo (which may be substituted by $C_{1-6}$ alkyl) and -A-R wherein

A is oxygen or sulfur, and

R is saturated or unsaturated $C_{1-6}$ hydrocarbon (which may be substituted by halogen, phenyl and/or phenoxy), or aryl containing not more than ten members and two or less thereof may be nitrogen (which may be substituted by halogen, cyano, $C_{1-6}$ haloalkyl and/or nitro);

n is zero or an integer of 1 to 3; and

m is an integer of 1 to 5.

13. A process for the preparation of a compound having the formula:

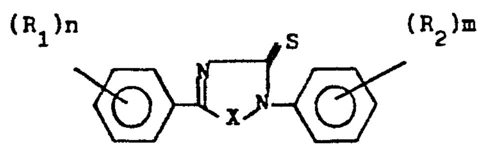

which comprises reacting a compound having the formula:

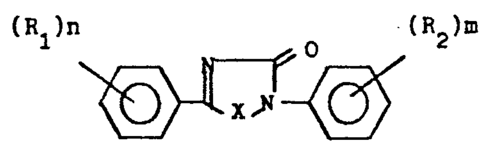

with Lawesson's Reagent having the formula:

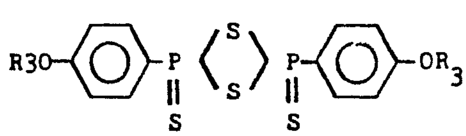

wherein X is oxygen or sulfur;

$R_1$ is same or different substituent(s) selected from the group consisting of halogen, $C_{1-3}$ alkyl, $C_{1-3}$ haloalkyl and $C_{1-3}$ alkoxy;

$R_2$ is same or different substituent selected from the group consisting of halogen, saturated or unsaturated $C_{1-6}$ hydrocarbon (which may be substituted by halogen), $C_{1-6}$ alkoxycarbonyl, $C_{1-6}$ alkyl substituted amino, phenylazo (which may be substituted by $C_{1-6}$ alkyl) and -A-R wherein

A is oxygen or sulfur, and

R is saturated or unsaturated $C_{1-6}$ hydrocarbon (which may be substituted by halogen, phenyl and/or phenoxy), or aryl containing not more than ten members and two or less thereof may be nitrogen (which may be substituted by halogen, cyano, $C_{1-6}$ haloalkyl and/or nitro); .

$R_3$ is $C_{1-6}$ alkyl;

n is zero or an integer of 1 to 3; and

m is an integer of 1 to 5.

14. A process for the preparation of a compound having the formula:

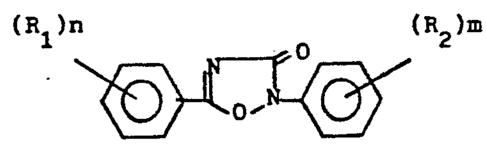

which comprises reacting a compound having the formula:

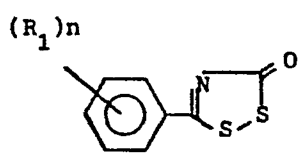

with a compound having the formula

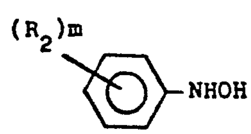

in the presence of a trivalent phosphorus compound

wherein $R_1$ is same or different substituent(s) selected from the group consisting of halogen, $C_{1-3}$ alkyl, $C_{1-3}$ haloalkyl and $C_{1-3}$ alkoxy;

$R_2$ is same or different substituent selected from the group consisting of halogen, saturated or unsaturated $C_{1-6}$ hydrocarbon (which may be substituted by halogen), $C_{1-6}$ alkoxycarbonyl, $C_{1-6}$ alkyl substituted amino, phenylazo (which may be substituted by $C_{1-6}$ alkyl) and -A-R wherein

A is oxygen or sulfur, and

R is saturated or unsaturated $C_{1-6}$ hydrocarbon (which may be substituted by halogen, phenyl and/or phenoxy), or aryl containing not more than ten members and two or less thereof may be nitrogen (which may be substituted by halogen, cyano, $C_{1-6}$ haloalkyl and/or nitro);

n is zero or an integer of 1 to 3; and

m is an integer of 1 to 5.